# EUROPEAN PATENT APPLICATION

(11) **EP 1 894 578 A1**
(43) Date of publication of application: **05.03.2008**
(21) Application number: 06122275.8
(22) Date of filing: 13.10.2006
(51) Int. Cl.: A61L 9/05, A61L 9/012, A61L 2/18, A45D 40/00, C11D 17/00, E03D 9/00

(54) **Method of applying a pasty composition for sanitary ware**

(30) Priority: 01.09.2006 EP 06120005
(71) Applicant: The Procter and Gamble Company, Cincinnati, Ohio 45202 (US)
(72) Inventor: Evers, Marc François Theophile, 1853, Strombeek-Bever (BE); Garmyn, Wim Peter Agnes, 2018, Antwerpen (BE); Gerlach, Christian Gerhard Friedrich, 1060, St. Gilles (Bruxelles) (BE); Höfte, Paulus Antonius Augustinus, 9830, St. Martens Latem (BE); Scialla, Stefano, 00128, Rome (IT)
(74) Representative: Kellenberger, Jakob

(57) **Abstract**

The present invention is directed to a method of applying a pasty composition for cleaning and/or deodorizing a sanitary ware, wherein the composition is suitable for direct application to the surface of the sanitary ware.

## Description

### TECHNICAL FIELD

The present invention relates to pasty compositions for cleaning and/or deodorizing sanitary ware such as toilet bowls, urinals, and sinks. More specifically, the present invention is directed to a method of applying a pasty composition for cleaning and/or deodorizing a sanitary ware, wherein the composition is suitable for direct application to the surface of the sanitary ware.

### BACKGROUND OF THE INVENTION

Compositions for cleaning and/or deodorizing sanitary ware such as toilets or urinals have long been sought by consumers, in both residential and commercial environments. In an attempt to meet the demand for sanitary cleaning compositions or deodorizers, numerous products have been developed and are presently available in the martketplace. In general, existing products are sold as solids, liquids or gel to provide the desired effect. In the context of toilet bowls, it is known to use sanitary agents in toilet hang-in baskets, which are attached through a holder to the toilet bowl edge, whereby the sanitary agent is released with each flushing action. Such sanitary agents are known in the form of solid blocks and recently also in gel form.
In spite of the long-lasting benefit provided by such products, the main disadvantage or inconvenience felt by the consumers occurs while replacing or re-filling by hand known sanitary agent for subsequent use. Those manipulations may be perceived as highly unhygienic and time-consuming. Also, the presence of known hanging devices baskets may disturb intermediary cleaning activities.
Other well known sanitary cleaning products are available in liquid form and packaged in squeezable bottles. Such liquid cleaning products however do only perform their cleaning action for a limited period.

Partial solutions to these drawbacks have been provided with for example in EP-A2-1 258 571 and WO 03/043906 which disclose dispensing devices for sanitary agent and method of applying thereof. The above-described dispensing devices are however used in such a way that they do not come into contact with the sanitary object. Although the disclosed method is claimed to be more hygienic to the user, the method does not allow for precise and proper application of pasty compositions to the treated sanitary object. Also, the disclosed method put the additional constraint to the user that any contact of the dispensing device to the treated surface should be carefully avoided.

It is therefore an objective of the present invention to provide a method of applying a pasty composition for cleaning and/or deodorizing a sanitary ware, wherein the composition is suitable for direct application to the surface of the sanitary ware, and which overcomes the above-mentioned drawbacks.

It has now been found that the above objective can be met by providing a method according to the present invention.

Advantageously, the method according to the present invention allows for precise and controlled application of pasty composition to the treated surface while remaining fully hygienic to the user, as the operator's hands are kept remote from potentially unhygienic portions of the treated sanitary ware.

A further advantage associated with the method according to the present invention is that it greatly improves usage convenience, and may be easily applied in both residential and commercial establishments. It is still a further advantage that the method according to the present invention is capable of providing long-term and sustained cleaning and/or air freshening benefits, while providing excellent mineral build-up prevention benefits and improved adhesion/dissolution profiles. Also, the method of the present invention contributes to provide the user with a more pleasant experience while operating sanitary ware cleaning tasks.

Other advantages and more specific properties of the method according to the present invention will be clear after reading the following description of the invention.

### SUMMARY OF THE INVENTION

The present invention relates to a method of applying a pasty composition for cleaning and/or deodorizing a sanitary ware, the composition being suitable for direct application to the surface of the sanitary ware, wherein the method comprises the steps of: a) providing a dispensing device **1** comprising a receiving container **2** for housing the pasty composition and a discharge nozzle **3** connected to said receiving container **2;** and b) dispensing the pasty composition through the discharge nozzle **3** while directly contacting the discharge nozzle **3** to the surface of the sanitary ware.

### BRIEF DESCRIPTION OF THE DRAWINGS

**FIG. 1** is a top perspective view of a dispensing device **1** for use in the method according to the present invention. The dispensing device comprises a receiving container **2** and a discharge nozzle **3** connected to said receiving container **2.**
**FIG. 2** is a top view of the dispensing device **1** represented in **FIG.1.**
**FIG. 3** is a side view of the dispensing device **1** represented in **FIG.1.**
**FIG. 4** is a top perspective view of an alternative dispensing device **1** for use in the method according to the present invention.

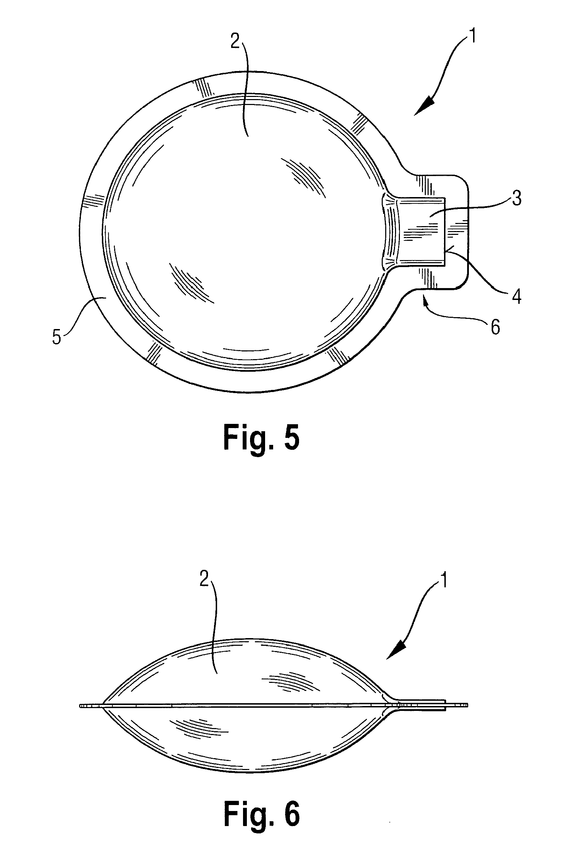

**FIG. 5** is a top view of the dispensing device **1** represented in **FIG.4**.
**FIG. 6** is a side view of the dispensing device **1** represented in **FIG.4**.

### DETAILED DESCRIPTION OF THE INVENTION

### PASTY COMPOSITION

The composition for use in the present invention is in the form of a pasty composition. By "pasty" composition, it is meant to encompass any of paste, gel, or cream composition. By "sanitary ware", it is meant herein any of toilet bowl, urinal, bathtub or sink. In the context of the present invention, the sanitary ware is preferably selected from toilet bowl and urinal, more preferably from toilet bowl.

The viscosity measurements mentioned in the present application have all been obtained with a TA Instruments/Advanced rheometer AR 1000, plate-plate system with stainless steel flat plate having a diameter of 40 mm, at a temperature of 20°C with a gap setting of 1000 microns, and at a shear rate of 25 s⁻¹.

Accordingly, the viscosity of the pasty composition for use in the present invention is preferably at least 5.000 mPas when measured with a TA Instruments/Advanced rheometer AR 1000 at 20°C. Typically, the viscosity of the composition according to the invention is comprised between 5.000 mPas and 500.000 mPas, preferably between 10000 mPas and 200.000 mPas, more preferably between 15.000 mPas and 150.000 mPas, and most preferably between 25.000 mPas and 100.000 mPas.

A pasty composition having a viscosity of at least 5.000 mPas, when measured with an AR 1000 Rheometer at 20°C, is believed to ensure better surface coverage of the applied composition to the treated surface. Also, viscous compositions, as herein described, are allowed to act on the surface more intimately than non-viscous liquid compositions and typically result in better cleaning and/or deodorizing performances.

The compositions for use in the present invention are advantageously provided with adhesiveness properties. The compositions herein are indeed capable of strongly and instantaneously adhering to the treated sanitary ware surface. The obtained adhesion shall typically be sufficient to prevent such pasty composition to be entirely detached under pressure exercised by the water flush. In contrast, the composition according to the present invention should only progressively be detached from the treated surface. Typically, the pasty composition shall be integrally washed away only after a large number of rinsing actions. Generally, the composition as described herein shall only be integrally rinsed-off after at least 30, preferably after at least 50, more preferably after at least 70, most preferably after between 80 and 200 rinse cycles.

### OPTIONAL INGREDIENTS

### Cationic surfactant

According to the present invention, the pasty composition for use herein may comprise a cationic surfactant as an optional but preferred ingredient. Suitable cationic surfactants for use herein may be selected from any cationic surfactant commonly known in the art.

Suitable cationic surfactants to be used herein include derivatives of quaternary ammonium, phosphonium, imidazolium, sulfonium compounds, and mixtures thereof. Preferred cationic surfactants for use herein are derivatives of quaternary ammonium. Accordingly, suitable cationic surfactants for use herein include, but are not limited to quaternary ammonium compounds wherein one or two of the hydrocarbon groups linked to nitrogen are a saturated or partially unsaturated, linear or branched alkyl group of 6 to 30 carbon atoms, preferably of 10 to 25 carbon atoms, and more preferably of 12 to 20 carbon atoms, and wherein the other hydrocarbon groups (i.e. three when one hydrocarbon group is a long chain hydrocarbon group as mentioned hereinbefore or two when two hydrocarbon groups are long chain hydrocarbon groups as mentioned hereinbefore) linked to the nitrogen are independently substituted or unsubstituted, linear or branched, alkyl chain of from 1 to 4 carbon atoms, preferably of from 1 to 3 carbon atoms, and more preferably are methyl groups.

Other suitable cationic surfactants for use in the method of the present invention include trimethyl quaternary ammonium compounds like myristyl trimethylsulfate, cetyl trimethylsulfate and/or tallow trimethylsulfate, and mixtures thereof. Such trimethyl quaternary ammonium compounds are commercially available from Hoechst, or from Albright & Wilson under the trade name EMPIGEN CM®. Other cationic surfactants useful herein are also described in U.S. Patent 4,228,044, Cambre, issued October 14, 1980.

Particularly preferred cationic surfactants for use herein are selected from the group consisting of diethyl ester hydroxyethyl methyl ammonium methyl (DEEHMAMS), wherein the hydrocarbon groups linked to the carbonyls are preferably a saturated, linear or branched alkyl group of 16 to 18 carbon atoms; diethyl ester dimethyl ammonium chloride (DEEDMAC), wherein the hydrocarbon groups linked to the carbonyls are preferably a saturated, linear or branched alkyl group of 16 to 18 carbon atoms, preferably an alkyl group of 18 carbon atoms; triethanol amine ester methyl ammonium methyl sulphate (TEEMAMS), wherein the hydrocarbon groups linked to the carbonyls are preferably a saturated or unsaturated, linear or branched alkyl group of 16 to 18 carbon atoms, more preferably an unsaturated, linear alkyl group of 17 carbon atoms; hydroxyethyl dimethyl ammonium chloride, wherein the hydrocarbon group linked to nitrogen is preferably a saturated, linear or branched alkyl group of 12 to 14 carbon atoms; hydroxyethyl dimethyl ammonium chloride, wherein the hydrocarbon group linked to nitrogen is preferably a saturated, linear or branched alkyl group of 8 to 10 carbon atoms; lauryl methyl bis hydroxyethyl ammonium chloride; dimethyl benzalkonium chloride, wherein the hydrocarbon group linked to nitrogen is preferably a saturated or unsaturated, linear or branched alkyl group of 12 to 18 carbon atoms, and mixtures thereof.

More preferably, the cationic surfactant is selected from the group consisting of DEEHMAMS, DEEDMAC, TEEMAMS, dimethyl benzalkonium chloride, and mixtures thereof.

Preferably, the composition for use in the present invention provides adequate adhesion without requiring additional use of adhesion promoters or tackifiers.

### Acidic compound

As another optional, but highly preferred ingredient, the pasty composition for use herein further comprises an acidic compound. According to the present invention, the pasty composition may typically comprise at least 5%, preferably at least 10%, more preferably at least 15% by weight of said composition of said acidic compound.

Suitable acidic compounds for use herein may be selected from any commonly known organic or inorganic acidic compounds, and mixtures thereof. Suitable organic acidic compounds for use herein include but are not limited to citric acid, maleic acid, adipic acid, oxalic acid, malic acid, succinic acid, tartaric acid, salicylic acid, aspartic acid, glutaric acid, malonic acid, and mixtures thereof. As a way of example, suitable inorganic acidic compounds comprise but are not limited to sulphamic acid, phosphoric acid, nitric acid, sulphonic acid, sulphuric acid, hydrochloric acid, or their salts or mixtures thereof.

Other acidic compounds useful herein include commonly known polymeric acids. Suitable polymeric acids for use herein comprise, but are not limited to polyacrylic polymers or acrylic-maleic copolymers, which are available e.g. from BASF under the tradenames Sokalan® CP5 or CP7 or CP9. Also useful herein are polyacrylic phosphono end group polymers or acrylic-maleic phosphono end group copolymers (available e.g. from Rohm &Haas under the tradenames Acusol® 420 or 470 or 425); and polyacrylic sulphono end group polymers or acrylic-maleic sulphono end group copolymers.

Preferably, acidic compound for use in the present invention is selected from acidic compounds in solid form. In an even more preferred embodiment, the acidic compound is in granular/powder form. Preferred acidic compounds for use herein include but are not limited to citric acid, maleic acid, and sulphamic acid. In a very preferred execution, the composition of the present invention comprises citric acid.

### Tackifying system

The composition for use in the present invention may optionally comprise a tackifier. Suitable tackifiers for use herein may be selected from any tackifier commonly known in the art, the choice of which is well within the capability of those skilled in the art. Suitable tackifiers for use in the compositions of the present invention may typically be defined as long or long-chained organic molecules, which are at least partly hydrophilic, and wherein the hydrophilic part of the tackifier interacts at least in part with water molecules and becomes tacky.
As a way of example suitable tackifiers for use herein include but are not limited to polyethylene glycol, polywax, cellulose, particularly sodium carboxymethyl cellulose, hydroxyethyl cellulose, hydroxypropyl cellulose, or polysaccharides such as xanthan gum, agar agar, gellan gum, acacia gum, carob bean flour, or guar gum or starch. It is also possible to use polymers such as polyacrylates, polyvinyl alcohols, polyvinyl pyrrolidones, and mixtures thereof. Suitable tackifiers may also include alginates, diurethanes, gelatins, pectines, oleyl amines, alkyl dimethyl amine oxides, or alkyl ether sulfates.
Preferred tackifiers are selected from the group consisting of short chain alcohols, glycols, polyglycols, glycerines, and mixtures thereof. More preferably, the tackifier is a short chain alcohol such as ethanol or isopropanol.

### Additional surfactants

As another preferred optional ingredient, the composition described herein may comprise additional surfactants. The presence of said surfactants in the compositions herein may allow providing compositions with desired viscosity by appropriately choosing surfactants and levels thereof.

Virtually any surfactant known in the art may be used as additional surfactant. Suitable surfactants may be advantageously selected from the group consisting of anionic, nonionic, cationic, amphoteric, zwiterrionic surfactants, and mixtures thereof. It is preferred to use anionic surfactant in the composition according to the present invention.

Accordingly, the compositions for use in the present invention comprise up to 50%, preferably of from 0.1% to 20%, more preferably of from 1% to 10%, and most preferably of from 1% to 5% by weight of the total composition of said additional surfactant, or mixtures thereof.

Suitable nonionic surfactants to be used herein are alkoxylated fatty alcohol nonionic surfactants that can be readily made by condensation processes that are well known in the art. Indeed, a great variety of such alkoxylated fatty alcohols are commercially available which have very different HLB values. Preferred nonionic surfactants for use in the present invention are the condensation products of ethylene oxide with alcohols having a straight alkyl chain, having from 6 to 22 carbon atoms, wherein the degree of ethoxylation is from 1 to 15, preferably from 5 to 12. Such suitable nonionic surfactants are commercially available from Shell, for instance, under the trade name Dobanol^{®} or from Shell under the trade name Lutensol^{®}.

Suitable amphoteric surfactants to be used in the compositions for use in the present invention include amine oxides having the following formula R₁R₂R₃NO wherein each of R1, R2 and R3 is independently a saturated substituted or unsubstituted, linear or branched alkyl groups of from 1 to 30 carbon atoms, preferably of from 6 to 30 carbon atoms, more preferably of from 10 to 20 carbon atoms, and most preferably of from 8 to 18 carbon atoms. Preferred amine oxides for use herein are for instance natural blend C₈-C₁₀ amine oxides as well as C₁₂-C₁₆ amine oxides commercially available from Hoechst.

Suitable anionic surfactants herein include water soluble salts or acids of the formula ROSO₃M wherein R is preferably a C₁₀-C₂₄ hydrocarbyl, preferably an alkyl or hydroxyalkyl having a C₁₀-C₂₀ alkyl component, more preferably a C₁₂-C₁₈ alkyl or hydroxyalkyl, and M is H or a cation, e.g., an alkali metal cation (e.g., sodium, potassium, lithium), or ammonium or substituted ammonium (e.g., methyl-, dimethyl-, and trimethyl ammonium cations and quaternary ammonium cations, such as tetramethylammonium and dimethyl piperdinium cations and quaternary ammonium cations derived from alkylamines such as ethylamine, diethylamine, triethylamine, and mixtures thereof). Preferred anionic surfactants for use in the compositions herein are the alkyl benzene sulfonates, alkyl sulfates, alkyl alkoxylated sulfates, and mixtures thereof.

### Perfumes

The compositions for use in the present invention may comprise, as an optional ingredient, perfume ingredient selected from the group consisting of : a cyclic terpene/sesquiterpene perfume, such as eucalyptol, cedrol, pinocarveolus, sesquiterpenic globulul alcohol; linalo; tetrahydrolinalo; verdox (cyclohexadiyl 2 tetryl butyl acetate); 6,3 hexanol; and citronellol and mixtures thereof.

The compositions for use herein may typically comprise from 0.01% to 10%, preferably from 0.01% to 5%, more preferably from 0.01% to 1%, and most preferably from 0.01 % to 0.1% by weight of the total composition of said perfume ingredient.

### Solvents

As another optional ingredient, the composition herein may comprise one or more solvents or mixtures thereof. Solvents for use herein include all those known in the art for use in hard-surface cleaner compositions. Suitable solvents can be selected from the group consisting of: aliphatic alcohols, ethers and diethers having from about 4 to about 14 carbon atoms, preferably from about 6 to about 12 carbon atoms, and more preferably from about 8 to about 10 carbon atoms; glycols or alkoxylated glycols; glycol ethers; alkoxylated aromatic alcohols; aromatic alcohols; terpenes; and mixtures thereof. Aliphatic alcohols and glycol ether solvents are most preferred, particularly those with vapour pressure of about 0.05 mm Hg at 25°C and 1 atmosphere pressure (about 6.66 Pa).

Aliphatic alcohols, of the formula R-OH wherein R is a linear or branched, saturated or unsaturated alkyl group of from about 1 to about 20 carbon atoms, preferably from about 2 to about 15 and more preferably from about 5 to about 12, are suitable solvents. Suitable aliphatic alcohols are methanol, ethanol, propanol, isopropanol or mixtures thereof. Among aliphatic alcohols, ethanol and isopropanol are most preferred because of their high vapour pressure and tendency to leave no residue.

Suitable glycols to be used herein are according to the formula HO-CR1R2-OH wherein R1 and R2 are independently H or a C2-C10 saturated or unsaturated aliphatic hydrocarbon chain and/or cyclic. Suitable glycols to be used herein are dodecaneglycol and/or propanediol.

Particularly preferred glycol ethers have a terminal C3-C6 hydrocarbon attached to from one to three ethylene glycol or propylene glycol moieties to provide the appropriate degree of hydrophobicity and, preferably, surface activity. Examples of commercially available solvents based on ethylene glycol chemistry include mono-ethylene glycol n-hexyl ether (Hexyl Cellosolve®) available from Dow Chemical. Examples of commercially available solvents based on propylene glycol chemistry include the di-, and tri-propylene glycol derivatives of propyl and butyl alcohol, which are available from Arco under the trade names Arcosolv® and Dowanol®.

### Chelating agents

Another class of optional compounds for use herein includes chelating agents. Chelating agents may be incorporated in the compositions herein in amounts ranging up to 10.0%, preferably 0.01% to 5.0% by weight of the total composition.

Suitable phosphonate chelating agents to be used herein may include alkali metal ethane 1-hydroxy diphosphonates (HEDP), alkylene poly (alkylene phosphonate), as well as amino phosphonate compounds, including amino aminotri(methylene phosphonic acid) (ATMP), nitrilo trimethylene phosphonates (NTP), ethylene diamine tetra methylene phosphonates, and diethylene triamine penta methylene phosphonates (DTPMP). The phosphonate compounds may be present either in their acid form or as salts of different cations on some or all of their acid functionalities. Preferred phosphonate chelating agents to be used herein are diethylene triamine penta methylene phosphonate (DTPMP) and ethane 1-hydroxy diphosphonate (HEDP). Such phosphonate chelating agents are commercially available from Monsanto under the trade name DEQUEST^{®}.

Polyfunctionally-substituted aromatic chelating agents may also be useful in the compositions herein. See U.S. patent 3,812,044, issued May 21, 1974, to Connor et al. Preferred compounds of this type in acid form are dihydroxydisulfobenzenes such as 1,2-dihydroxy -3,5-disulfobenzene.

A preferred biodegradable chelating agent for use herein is ethylene diamine N,N'-disuccinic acid, or alkali metal, or alkaline earth, ammonium or substitutes ammonium salts thereof or mixtures thereof. Ethylenediamine N,N'-disuccinic acids, especially the (S,S) isomer, have been extensively described in US patent 4, 704, 233, November 3, 1987, to Hartman and Perkins. Ethylenediamine N,N'-disuccinic acid is, for instance, commercially available under the tradename ssEDDS^{®} from Palmer Research Laboratories.

Suitable amino carboxylates to be used herein include ethylene diamine tetra acetates, diethylene triamine pentaacetates, diethylene triamine pentaacetate (DTPA), N-hydroxyethylethylenediamine triacetates, nitrilotri-acetates, ethylenediamine tetrapropionates, triethylenetetraaminehexa-acetates, ethanol-diglycines, propylene diamine tetracetic acid (PDTA) and methyl glycine di-acetic acid (MGDA), both in their acid form, or in their alkali metal, ammonium, and substituted ammonium salt forms. Particularly suitable amino carboxylates to be used herein are diethylene triamine penta acetic acid, propylene diamine tetracetic acid (PDTA) which is, for instance, commercially available from BASF under the trade name Trilon FS^{®} or Trilon M^{®} and methyl glycine di-acetic acid (MGDA).

Further carboxylate chelating agents to be used herein include salicylic acid, aspartic acid, glutamic acid, glycine, malonic acid or mixtures thereof.

In addition to the above-mentioned optional ingredients, the composition for use in the present invention may also further comprise other components selected from the group of fragrances, thickeners, colorants, dyes, optical brighteners, builders, chelants, preservatives, solvents, buffering agents, radical scavengers, stabilizers, dye scavengers, adhesion promoters, rheology modifiers, bleaching systems, and mixtures thereof.

### DISPENSING DEVICE 1

As indicated above, the dispensing device **1** for use in the method according to the present invention comprises a receiving container **2** for housing the pasty composition as above-described and a discharge nozzle **3** connected to said receiving container **2.**

The receiving container **2** may have any suitable configuration, form or dimension for accommodating and housing a pasty composition. Suitable receiving container **2** for use herein may be of any overall shape including, but not limited to spherical, ovoid, square, triangular, rectangular, cylindrical, polygonal, trapezoidal, and combinations thereof. Preferably, the receiving container **2** for use herein has an overall shape selected from the group of ovoid, cylindrical, triangular, trapezoidal, and combinations thereof. Accordingly, the receiving container **2** is preferably in a form selected from the group of tubes, pouches, capsules, boxes, bottles, tottles, envelopes, thermoformed cups, thermoformed blisters, cans, bags, syringes, atomizers, aerosols, pressurized cans, piston-driven cans, and combinations thereof. More preferably, the receiving container **2** is in a form selected from the group of tubes, pouches, bottles, tottles, and combinations thereof.

In one preferred execution of the present invention, the receiving container **2** is made from a deformable or flexible material. By "deformable or flexible" material it is meant herein that the material is provided with a certain resiliency which renders the corresponding receiving container **2** capable of being temporarily or permanently upon deformed pressure application. According to such a preferred embodiment, the receiving container **2** is preferably in a form selected from the group of tubes, pouches, capsules, boxes, bottles, tottles, and combinations thereof. Typically, the corresponding receiving container **2** will then take the form of a squeezable container which upon pressure will cause the contained pasty composition to be expulsed from the receiving container **2** via the discharge nozzle **3** connected to said receiving container **2.**

Suitable flexible material for use herein may be easily recognized by those skilled in the art of packaging. As a way of example, suitable flexible material may be selected from the group consisting of heat sealable thermoplastic materials such as Polyethylene (PE), Low Density Polyethylene (LDPE), High Density Polyethylene (HDPE), Polypropylene (PP), Polyethylene Terephthalate (PET), Polyethylene Terephthalate-G (PETG), Polyvinyl Chloride (PVC), Polystyrene (PS), High Impact Polystyrene (HIPS), Polyvinylidene Chloride (PVDC); engineering grade plastics such as Acrylonitrile Butadiene Styrene (ABS), Polyoxymethylene (e.g. Delrin™, Celcon™), thermoplastic elastomers (TPE), and mixtures thereof.
Other suitable flexible material for use herein include aluminium, laminated structures such as e.g. carton, barrier carton, co-injected materials, barrier films or foils including metal foils (aluminum), sputtered or vaporized aluminum, Polyamides (Nylon), Teflon®, Ethyl Vinyl Alcohol (EVOH), Surlyn®, and mixtures thereof.

More preferably, the receiving container **2** is produced as a single piece plastics moulded article using any commonly known technique selected from the group of vacuum thermoforming, injection molding, extrusion blow molding, extrusion stretch blow molding, laminated tube or extruded tube forming, flexible film heat sealing, laminated carton board heat sealing packing techniques, and combinations thereof.

In another preferred execution of the present invention, the receiving container **2** is made from a non-flexible material such as metal, thermoplastics, PET, PETG, Polyethylene Naphthalate (PEN), PP, PE, and mixtures thereof. According to such preferred execution, the corresponding receiving container **2** is preferably in a form selected from the group of cans, atomizers, aerosols, pressurized cans, Airopack™, and combinations thereof. According to still another preferred execution, the receiving container may take the form of airless systems such as e.g. bag in cans, bag in bottle, delaminating bottles, bag in tube, tube in tube, piston in can, and combinations thereof

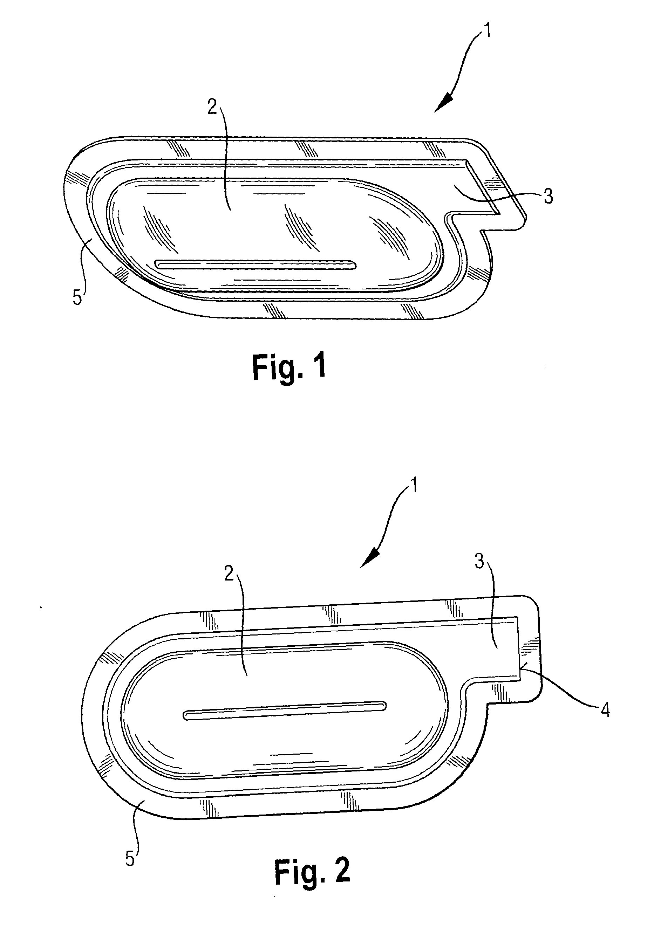

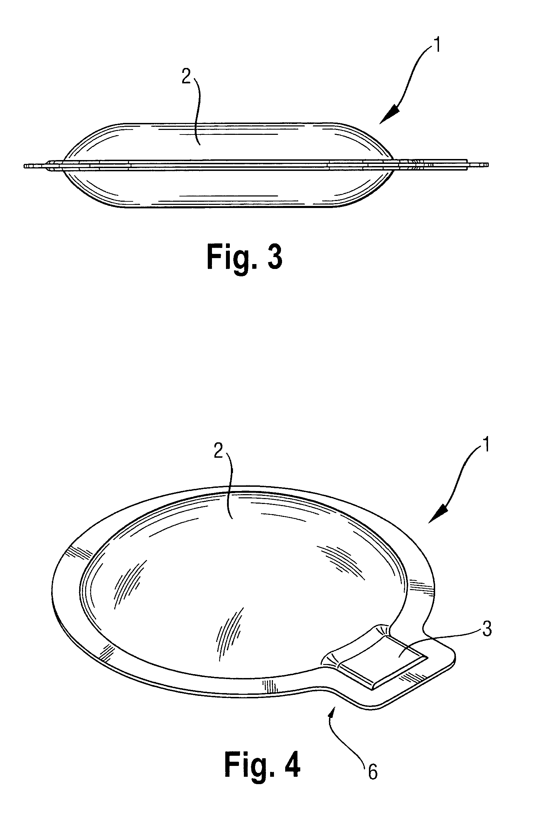

In a very preferred execution of the present invention, and as represented in **FIG.1** and **FIG.4,** the receiving container **2** is in a form of a squeezable container.

As above-mentioned, the dispensing device **1** for use herein further comprises a discharge nozzle **3** connected to the receiving container **2.** The discharge nozzle **3** may have any suitable configuration, form or dimension for accommodating the receiving container **2** and for allowing the pasty composition contained within the receiving container **2** to be dispensed via said discharge nozzle **3.** Accordingly, the discharge nozzle **3** defines a passageway for the pasty composition contained within the receiving container **2.**

Typically, the discharge nozzle **3** is an extension of the receiving container **2** and is either in free connection with the receiving container **2** or said discharge nozzle **3** is separated from the receiving container **2** via a temporary seal, preferably a rupturable seal (not represented). Suitable discharge nozzle **3** for use herein may be of any overall shape including, but not limited to spherical, ovoid, square, triangular, rectangular, cylindrical, polygonal, trapezoidal, and combinations thereof. Preferably, the discharge nozzle **3** for use herein has an overall shape selected from the group of cylindrical, trapezoidal, rectangular, and combinations thereof. In a preferred execution of the present invention, the discharge nozzle **3** for use herein is provided with a substantially rectangular shape, in a substantially flat configuration.

According to a preferred execution of the present invention, the length of the discharge nozzle **3** is of at least 0.5 cm, when said length corresponds to the distance between the end of the discharge nozzle **3** and the point where the discharge nozzle contacts the receiving container **2.** More preferably, the length of the discharge nozzle **3** is of at least 1 cm, even more preferably of at least 2 cm.

The discharge nozzle **3** for use herein is typically provided with a dispensing aperture **4,** which is preferably temporarily obstructed before the first use via a seal rupturable/openable upon pressure (not represented).

According to a preferred execution, the dispensing device **1** is further provided with a peripheral flange **5.** The peripheral flange **5** may be the result of the dispensing device **1** manufacturing process or may be intentionally included. Typically, the peripheral flange **5** will extend beyond the periphery of the receiving container **2** and the discharge nozzle **3.** As such, the peripheral flange **5** will improve rigidity and/or stability of the dispensing device **1.** According to a preferred embodiment, the portion of the peripheral flange **5** extending around the periphery of the discharge nozzle **3** will typically form a spatula **6.** The spatula **6** for use herein may help in dispensing the pasty composition in a more controlled and precise manner.

Preferably, the dispensing device **1** is provided as a lightweight and portable device. Typically, the dispensing device **1** for use herein may be squeezable upon manual pressure exercised by the fingers of a user.

### METHOD OF APPLYING A PASTY COMPOSITION

According to the present invention, it is provided a method of applying a pasty composition for cleaning and/or deodorizing a sanitary ware, the composition being suitable for direct application to the surface of the sanitary ware, wherein the method comprises the steps of: a) providing a dispensing device **1** comprising a receiving container **2** for housing the pasty composition and a discharge nozzle **3** connected to said receiving container **2;** and b) dispensing the pasty composition through the discharge nozzle **3** while directly contacting the discharge nozzle **3** to the surface of the sanitary ware.

By "directly contacting the discharge nozzle **3** to the surface of the sanitary ware", it is meant herein that the discharge nozzle **3** is physically contacted to the surface of the sanitary ware while said pasty composition being dispensed through the discharge nozzle **3.** As a result, both the pasty composition and the discharge nozzle **3** are directly contacted to the surface of the sanitary ware.

Preferably, the step of dispensing the pasty composition through the discharge nozzle **3** is operated by manually squeezing the receiving container **2.**
According to an alternative embodiment of the present invention, the step of dispensing the pasty composition is operated via a suitable actuating means including, but not limited to a trigger, a pushing button, a piston, a lever, or combinations thereof. Such dispensing action is particularly suitable when using a receiving container **2** in the form of a can, atomizer, aerosol, or pressurized can.

In the particular embodiment wherein the discharge nozzle **3** is provided with a peripheral flange **5,** the expression "discharge nozzle" for the purpose of step b) as above described should be understood as describing the combination of the discharge nozzle **3** and the peripheral flange **5** so that according to this particular embodiment, it is preferably the peripheral flange which is actually contacted with the surface of the sanitary ware.

The method for applying a pasty composition according to the present invention prevents the user from contacting by hand dirtied and unhygienic surfaces or objects, via using suitable dispensing device **1.** This benefit is particularly enhanced in the preferred execution wherein the discharge nozzle **3** has a length of at least at least 0.5 cm, preferably at least 1 cm, more preferably at least 2 cm. Also, the distance between the treated surface and the dispensing device **1** is believed to provide better ergonomics during the pasty composition application and virtually eliminates any psychological impression to operate in unhygienic environment.

Furthermore, the method according to the invention gives the user a simple and highly controlled way of applying the composition as the latter is allowed to choose the specific amount of composition which needs to be applied to the sanitary ware. By directly contacting the discharge nozzle **3** to the surface of the sanitary ware, the user is allowed to apply the pasty composition in a more precise and faster manner, which is highly appreciable in the context of sanitary ware cleaning and/or deodorizing operation. Also, the method of the invention provides the user the flexibility to choose exactly the location(s) of the sanitary ware where the composition needs to be applied.
This overall provides the consumer with a totally new, hygienic and exciting toilet care experience.

### EXAMPLES

These following compositions were made comprising the listed ingredients in the listed proportions (weight %). The examples herein are met to exemplify the present invention but are not necessarily used to limit or otherwise define the scope of the present invention. Compositions I to X are compositions according to the present invention.

| Ingredients: (% by weight) | I | II | III | IV | V |
|---|---|---|---|---|---|
| DEEHMAMS | 7 | 7.5 | 8.3 | - | - |
| Uniquat® | 13 | 22.5 | 16.7 | 13 | - |
| TEEMAMS | - | - | - | - | - |
| DEEDMAC | - | - | - | 7 | - |
| CMBHAC | - | - | - | - | - |
| Amineoxide | - | - | - | - | - |
| CnAE3S | - | - | - | - | 25 |
| Alkanolamide | - | - | - | - | - |
| Citric S40 | 30 | 18 | 25 | 30 | 18 |
| Sodiumsulphate | 45 | 50 | 43 | 46 | 30 |
| Perfume | 2 | 2 | 2 | 2 | 2 |
| PEG 9000MW | - | - | 5 | - | - |
| PVP | - | - | - | - | - |
| Isopar® M | - | - | - | - | 4 |
| Glycerol | 2 | - | - | - | - |
| Waters & Minors | Up to 100 | | | | |

DEEHMAMS is a Diethyl Ester Hydroxyethyl Methyl Ammonium Methyl Sulphate, supplied by Stepan under the tradename Stepantex VK90®.
Uniquat® is a C12-C18 Benzalkonium chloride, supplied by Lonza.
TEEMAMS is a Triethanol Amine Ester Methyl Ammonium Methyl Sulphate, supplied by Degussa under the tradename Rewoquat V3620®.

| Ingredients: (% by weight) | VI | VII | VIII | IX | X |
|---|---|---|---|---|---|
| DEEHMAMS | 7.5 | - | - | 15 | - |
| Uniquat® | 22.5 | 16.7 | - | 15 | - |
| TEEMAMS | - | 8.3 | 7.5 | - | - |
| DEEDMAC | - | - | - | - | - |
| CMBHAC | - | - | 22.5 | - | - |
| Amineoxide | 3 | - | - | - | - |
| CnAE3S | - | - | - | - | - |
| Alkanolamide | - | - | - | - | 35 |
| Citric S40 | 18 | 39 | 29 | 18 | - |
| Sodiumsulphate | 45 | 28 | 28 | 43 | 37 |
| Perfume | 2 | 4 | 4 | 2 | 2 |
| PEG 9000MW | - | 2 | 5 | - | - |
| PVP | - | - | 1 | 2 | - |
| Isopar® M | - | - | - | - | - |
| Glycerol | - | - | - | - | - |
| Waters & Minors | Up to 100 | | | | |

DEEDMAC is Diethyl Ester Dimethyl Ammonium Chloride, supplied by Degussa under the tradename Rewoquat V3282®.
CMBHAC is Coconut methyl bishydroxyethyl ammoniumchloride, supplied by AKZO-Nobel.
Amine oxide is AO728®, supplied by Tomah.
CnAE3S is Coconut alkyl ethoxylated (3EO) sulphate supplied by Univar Benelux under the tradename Empicol® ESC 70/U.

Alkanolamide is Peg-50 Hydrogenated Palmamide supplied by AKZO-Nobel under the tradename Ethomid® HP/50.
Citric acid S40 is supplied by Jungbunzlauer.
PEG is polyethylene glycol, supplied by BASF.
PVP is polyvinyl pyrrolidone, supplied by BASF.
Isopar® M is paraffin supplied by Exxon.
Glycerol is supplied by P&G Chemicals.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A method of applying a pasty composition for cleaning and/or deodorizing a sanitary ware, said composition being suitable for direct application to the surface of said sanitary ware, wherein the method comprises the steps of:
a) providing a dispensing device **1** comprising a receiving container **2** for housing said pasty composition and a discharge nozzle **3** connected to said receiving container **2;** and
b) dispensing said pasty composition through said discharge nozzle **3** while directly contacting said discharge nozzle **3** to the surface of said sanitary ware.

2. A method according to any of claims 1, wherein the length of said discharge nozzle **3** is of at least 0.5 cm, preferably at least 1 cm, more preferably at least 2 cm.

3. A method according to any of claims 1 or 2, wherein said receiving container **2** is made of a deformable material.

4. A method according to claim 3, wherein said receiving container **2** is made of heat sealable thermoplastic materials such as Polyethylene, Polypropylene, Polyethylene Terephthalate, Polyvinyl Chloride, Polystyrene (PS), Polyvinylidene Chloride, and mixtures thereof.

5. A method according to any of the preceding claims, wherein said receiving container **2** is in a form selected from the group of tubes, pouches, capsules, boxes, bottles, tottles, envelopes, thermoformed cups, thermoformed blisters, cans, bags, syringes, atomizers, aerosols, pressurized cans, piston-driven cans, and combinations thereof.

6. A method according to claim 5, wherein said receiving container **2** is in the form selected from the group of tubes, pouches, bottles and tottles, preferably a tottle.

7. A method according to any of the preceding claims, wherein said dispensing device **1** is further provided with a peripheral flange **5.**

8. A method according to claim 7, wherein the portion of said peripheral flange **5** extending around the periphery of said discharge nozzle **3** forms a spatula **6.**

9. A method according to any of the preceding claims, wherein said pasty composition is dispensed through said discharge nozzle **3** by manual action.

10. A method according to any of the preceding claims, wherein the viscosity of said composition is at least 5.000 mPas, when measured with a TA Instruments/Advanced rheometer AR 1000 at a temperature of 20°C with a gap setting of 1000 microns, and at a shear rate of 25 s⁻¹.

11. A method according to any of the preceding claims, wherein the viscosity of said composition is comprised between 5.000 mPas and 500.000 mPas, preferably between 10000 mPas and 200.000 mPas, more preferably between 15.000 mPas and 150.000mPas, and most preferably between 25.000 mPas and 100.000 mPas, when measured with a TA Instruments/Advanced rheometer AR 1000 at a temperature of 20°C with a gap setting of 1000 microns, and at a shear rate of 25 s⁻¹.

12. A method according to any of the preceding claims, wherein said composition comprises a cationic surfactant, preferably selected from the group consisting of DEEHMAMS, DEEDMAC, TEEMAMS, dimethyl benzalkonium chloride, and mixtures thereof.

13. A method according to any of the preceding claims, which further comprises components selected from acidic compounds, tackifiers, fragrances, thickeners, colorants, dyes, preservatives, additional surfactants, solvents, dye scavengers, adhesion promoters, bleaching systems, and mixtures thereof.

14. A method according to any of the preceding claims, wherein said composition is in a form selected from of a gel, a paste, and a cream.

15. A method according to any of the preceding claims, wherein said sanitary ware is a toilet bowl.

16. A method according to claim any of the preceding claims, whereby said composition is washed away only after at least 30, preferably after at least 50, more preferably after at least 70, most preferably after between 80 and 200 rinse cycles.
